# EUROPÄISCHE PATENTSCHRIFT

(11) **EP 1 871 304 B1**
(45) Veröffentlichungstag und Bekanntmachung des Hinweises auf die Patenterteilung: **07.09.2011**
(21) Anmeldenummer: 05817363.4
(22) Anmeldetag: 28.11.2005
(51) Int. Cl.: A61F 2/38

(54) **GELENKPROTHESE MIT ZWISCHENELEMENT MIT UNTERSCHIEDLICH GESTALTETEN GLEITFLÄCHEN**
JOINT PROSTHESIS COMPRISING AN INTERMEDIATE ELEMENT HAVING DIFFERENT SLIDING SURFACES
PROTHESE ARTICULAIRE COMPORTANT DES ELEMENTS INTERMEDIAIRES PRESENTANT DES SURFACES DE GLISSEMENT DE FORMES DIFFERENTES

(30) Priorität: 02.03.2005 DE 102005009496
(43) Veröffentlichungstag der Anmeldung: 02.01.2008
(73) Patentinhaber: Mathys AG Bettlach, 2544 Bettlach (CH)
(72) Erfinder: FANKHAUSER, Christoph, CH-4500 Solothurn (CH); SUPPER, Walter, CH-2540 Grenchen (CH); DELFOSSE, Daniel, CH-3303 Jegenstorf (CH); WYSS, Tobias, CH-3122 Kehrsatz (CH)
(74) Vertreter: Körfer, Thomas
(86) Internationale Anmeldenummer: PCT/EP2005/012700
(87) Internationale Veröffentlichungsnummer: WO 2006/092167

(56) Entgegenhaltungen:
- DE-A1- 19 529 824
- US-A- 5 413 604
- US-A- 5 871 543
- US-B1- 6 190 415

## Beschreibung

Die Erfindung betrifft eine Gelenkprothese zum Ausbilden einer Gelenkverbindung zwischen einem Paar menschlicher oder tierischer Knochen.

Beispielsweise ist aus der WO 98/02116 eine Kniegelenk-Endoprothese bekannt, welche ein Femurteil und ein Tibiateil umfaßt. Zumindest die Oberfläche des medialen Kondylenabschnitts der Femurkomponente ist dabei annähernd gleich gestaltet wie eine mediale Kavität in der Oberfläche der Tibiakomponente, so daß sich ein vollflächiger Kontakt ergibt. Die Tibiakomponente umfaßt dabei ein Basisteil und eine darauf lösbar und verschieblich fixierte Gleitkomponente.

Weiterhin ist aus der EP 0 765 645 A2 eine ähnliche Kniegelenk-Endoprothese bekannt. Bei dieser soll durch eine Vergrößerung des coronaren Radius der Gleitfläche der Femurkomponente in anterio-posteriorer Richtung eine Verbesserung der Bewegungsabläufe erzielt werden.

Die US-A-5 413 604 beschreibt eine Kniegelenk-Endoprothese, die als totaler Knieersatz bei einem nur unzureichend vorhandenem vorderem Kreuzband verwendet wird. Die beschriebene Kniegelenk-Endoprothese umfasst eine femorale Komponente, die eine laterale und eine mediale Kondyle umschließt, eine Tibiakomponente, die ein Zwischenelement mit Gleitflächen für die Kondylen der femoralen Komponente aufnimmt und eine Drehvorrichtung, die eine Drehbewegung zwischen der femoralen Komponente und der Tibiakomponente um eine Achse, die parallel zum Tibiaknochen durch die laterale Gleitfläche verläuft, ermöglicht. Die Drehvorrichtung erlaubt eine anterior-posteriore Verschiebung der medialen Kondyle gegenüber der lateralen Kondyle in einer anterioren Richtung bei der Beugung des Knies und in eine posteriore Richtung während der Streckung des Knies. Um diese Verschiebung zu ermöglichen, ist die mediale Gleitfläche in Anterior-Posterior-Richtung lang gezogen ausgeformt. Die laterale Gleitfläche ist dagegen kreisrund bzw. sphärisch-konkav ausgebildet und somit nahezu kongruent zur darin gelagerten lateralen Kondyle, sodass nahezu keine Verschiebung der lateraler Kondyle in der lateraler Gleitfläche möglich ist.

Dokument US-B1-6 190 415 offenbart eine Kniegelenk-Endoprothese, die ebenfalls aus einer Femurkomponente, einer Tibiakomponente und mindestens einem Zwischenelement aufgebaut ist. Die Femurkomponente besitzt eine mediale und eine laterale Kondylenfläche, die die Kondyle nach posterior ca. 160 Grad gegenüber der Knochenachse umfasst. In die Tibiakomponente sind zwei Kunststoffeinsätze eingelegt, die einerseits mit der medialen anderseits mit der lateralen Kondyle eine Gleitpaarung bilden. Die, den Kondylen zugewandte, Gleitfläche der Kunststoffeinsätze ist konkav ausgeformt, sodass sie die konvex geformten Kondylen mit genügendem Spielraum aufnehmen können. Beide Zwischenelemente sind unabhängig beweglich auf der Tibiakomponente aufgesetzt. Die, der Tibiakomponente zugewandte Oberfläche des medialen Zwischenelements ist konvex geformt und greift in eine konkave Oberfläche der Tibiakomponente ein. Die entsprechende Oberfläche des lateralen Zwischenelements ist konkav ausgeformt und ist in einem konvexen Bereich der Tibiakomponente aufgesetzt.

Nachteilig bei den aus den obengenannten Druckschriften bekannten Endoprothesen ist insbesondere, daß bedingt durch die nicht naturgetreue Form der Gleitkomponenten die physiologischen Kniegelenksfunktionen bei Bewegung und Belastung nicht befriedigend erfüllt werden. Insbesondere treten örtliche Belastungsspitzen in Folge von Linien- oder Punktberührungen auf. In der Folge können Bewegungsstörungen, ungleichmäßige Abnützung der Prothese, Luxationen oder Lockerungen der tibialen oder femoralen Komponente der Endoprothese auftreten.

Der Erfindung liegt demnach die Aufgabe zugrunde, eine Gelenkprothese zu schaffen, welche möglichst naturgetreue physiologische Bewegungsabläufe ermöglicht, ohne daß es zu Fehlstellungen oder Instabilität und nachfolgenden Fehlfunktionen oder Zerstörung der Prothese kommen kann.

Die Aufgabe wird durch eine Gelenkprothese gemäß den kennzeichnenden Merkmalen des Hauptanspruchs in Verbindung mit den gattungsbildenden Merkmalen gelöst.

Der Erfindung liegt die Erkenntnis zugrunde, daß es vorteilhaft ist, wenn eine laterale Gleitfläche und eine mediale Gleitfläche des zwischen Femur- und Tibiakomponente angeordneten Zwischenelements unterschiedliche sagittale Querschnittsprofile aufweisen. Daraus resultiert ein höherer Komfort durch einen verbesserten Bewegungsbereich (range of motion) und deutlich weniger stark belastete Weichteilstrukturen für den prothetisch versorgten Patienten.

Vorteilhafte Weiterbildungen der Erfindung sind durch die in den Unteransprüchen angegebenen Maßnahmen möglich.

Besonders vorteilhaft ist dabei, daß die mediale Gleitfläche stets konkav gewölbt ist, während die laterale Gleitfläche zumindest teilweise, insbesondere im dorsalen Kompartiment, konvex oder eben, jedoch dorsal-distal abfallend geformt ist.

Vorteilhafterweise ist die laterale Gleitfläche in Zonen aufgeteilt, welche unterschiedliche Krümmungsradien aufweisen.

Die Krümmungsradien der einzelnen Zonen gehen vorteilhafterweise an Wendepunkten stetig in die Krümmungsradien der benachbarten Zonen über.

Weiterhin ist von Vorteil, daß das Zwischenelement aus Polymeren, keramischen und/oder metallischen Werkstoffen hergestellt sein kann. Besonders bevorzugt ist dabei das Polyethylen UHMWPE.

Die Erfindung wird nachfolgend anhand von Ausführungsbeispielen unter Bezugnahme auf die Zeichnung näher beschrieben. In der Zeichnung zeigen:
- Fig. 1A: ein Ausführungsbeispiel für eine Kniegelenk- Endoprothese gemäß dem Stand der Technik,
- Fig. 1B: ein Ausführungsbeispiel eines Zwischenelements einer Kniegelenk- Endoprothese einer schematischen Aufsicht,
- Fig. 2A-2D: Ausführungsbeispiele für anterio-posteriore Querschnittsformen in einer lateralen Schnittebene,
- Fig. 3: ein Ausführungsbeispiel der anterio- posterioren Querschnittsform in einer medialen Schnittebene, und
- Fig. 4A-B: eine schematische perspektivische und eine schematische perspektivische, teilgeschnitte- ne Ansicht des erfindungsgemäß ausgestalteten Zwischenelements gemäß Fig. 1B.

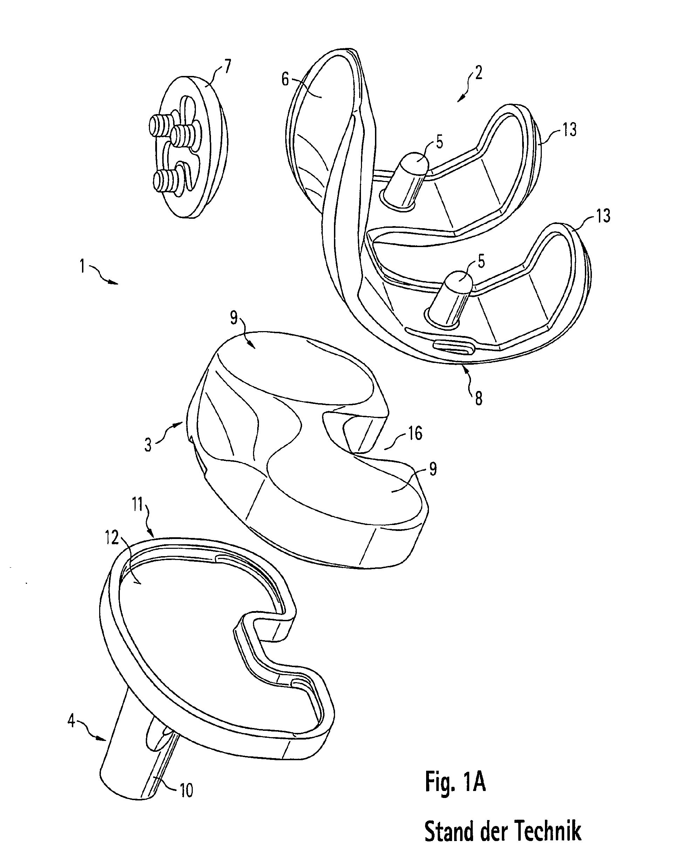

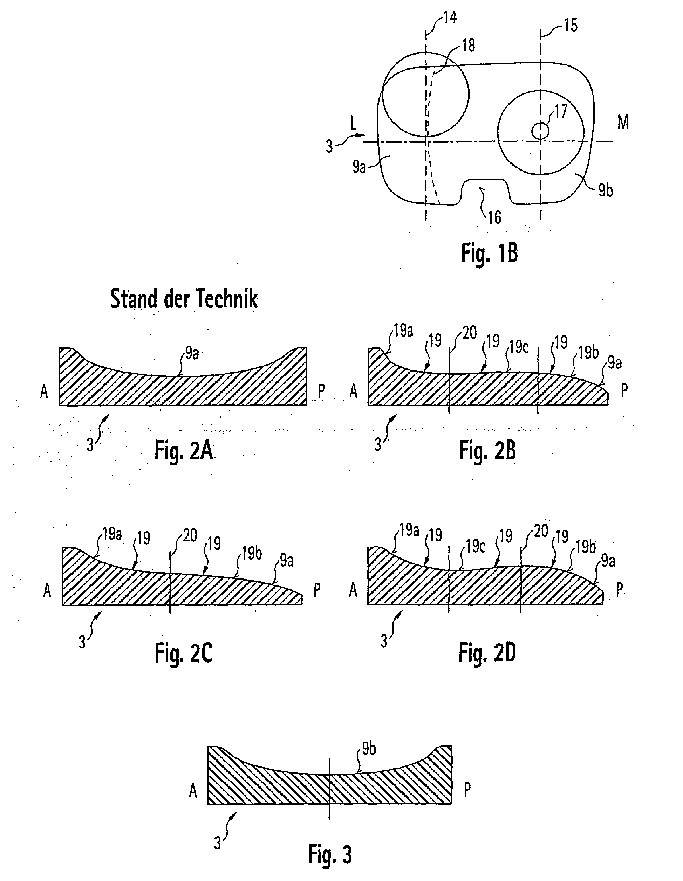

Bevor anhand der Fig. 2A bis 2D, Fig. 3 und Fig. 4A und 4B Ausführungsbeispiele eines Zwischenelements einer erfindungsgemäßen Kniegelenk-Endoprothese dargestellt wird, soll zunächst zur besseren Verständlichkeit der erfindungsgemäßen Maßnahmen anhand von Fig. 1A eine Kniegelenk-Endoprothese gemäß dem Stand der Technik in ihren wesentlichen Komponenten kurz erläutert werden.

Die Kniegelenk-Endoprothese 1 gemäß dem Stand der Technik weist eine Femurkomponente 2 auf, welche zur prothetischen Versorgung beider Kondylen des Oberschenkelknochens (Femur) ausgestaltet ist. Sie wird mittels zweier Zapfen 5 im Oberschenkelknochen verankert und weist eine anteriore Gleitfläche 6 für ein Patellaimplantat 7 sowie distalen Gleitflächen 8 auf, welche auf einem Zwischenelement 3 gleiten.

Das Zwischenelement 3 ersetzt die Funktion der Menisci des Kniegelenks und weist ebenfalls Gleitflächen 9 auf, welche mit den distalen Gleitflächen 8 der Femurkomponente 2 korrespondieren.

Das Zwischenelement 3 ist lösbar mit einer Tibiakomponente 4 verbunden, welche mit einem Schaft 10 in einen Schienbeinknochen implantiert wird und als Träger für das Zwischenelement 3 dient. Eine Ausnehmung 16 des Zwischenelements 3 greift dabei unter einen Rand 11, mit welchem die Tibiakomponente 4 versehen ist, welcher dafür sorgt, daß das Zwischenelement 3 nicht aus dem Kniegelenkspalt auswandern kann. Eine Anlagefläche 12 der Tibiakomponente 4 kann dabei eben ausgebildet sein, was die Herstellung vereinfacht.

Nach den vorbereitenden Resektionen von Femur und Tibia werden zunächst die Femurkomponente 2 und die Tibiakomponente 4 eingesetzt und dann ein auf die Situation angepaßtes Zwischenelement 3 in die Tibiakomponente 4 eingelegt.

Wie aus Fig. 1A ersichtlich, ist dabei die prothetische Versorgung beider Femur-Kondylen durch die dargestellte bikondyläre Kniegelenk-Endoprothese, 1 angestrebt. Das Zwischenelement 3 weist folglich für die beiden Kondylenfortsätze 13 der Femurkomponente 2 die bereits erwähnten Gleitflächen 9 auf, welche konkav in dem Zwischenelement 3 ausgebildet sind.

Eine derartige Ausbildung des Zwischenelements 3 kann jedoch die physiologischen Bewegungsabläufe im Gelenk nur sehr unzureichend reproduzieren. Insbesondere ist der Bewegungsbereich (sog. range of motion) im Vergleich zu einem natürlichen Kniegelenk stark eingeschränkt. Die tiefe Kniebeugung und die notwendige axiale Innenrotation des Schienbeins mit zunehmender Beugung sind eingeschränkt, als Folge einer partiellen Überbeanspruchung ligamentärer Strukturen, was das Zwischenelement 3 einer ungewollten und zu hohen Beanspruchung und dadurch erhöhter Abnutzung aussetzen kann.

Demgegenüber weist das Zwischenelement 3 der erfindungsgemäß ausgestalteten Kniegelenk-Endoprothese 1 gemäß den Fig. 1B, 2 und 3 davon abweichende Geometrien der Gleitflächen 9 auf.

In Fig. 1B ist eine schematische Aufsicht auf ein Zwischenelement 3 dargestellt, in welchem zwei sagittale Schnittebenen 14 und 15 markiert sind. Dabei ist die Schnittebene 14 lateral und die Schnittebene 15 medial relativ zur Positionierung des Zwischenelements 3 im Gelenk vorgesehen.

Erfindungsgemäß weist der mediale Sagittalschnitt jeweils eine identische, konkave Form gemäß Fig. 3 auf, während die lateralen Sagittalschnitte gemäß den Fig. 2A bis 2D unterschiedlich dazu ausgewählt werden können. Die in Fig. 2A bezeichnete Querschnittsform bezeichnet dabei den Stand der Technik, da sich dabei eine Gleitfläche 9 ergibt, welche sowohl medial wie auch lateral konkav ausgebildet ist (Fig. 2A in Verbindung mit Fig. 3).

Kombiniert man jedoch die mediale konkave Querschnittsform gemäß Fig. 3 mit einer der Querschnittsformen gemäß Fig. 2B bis 2D, erhält man jeweils ein Zwischenelement 3, welches bedingt durch die Verwendung konvexer oder zumindest ebener Krümmungsradien vollkommen andere, physiologisch der natürlichen Kniegelenksartikulation erheblich näherkommende Bewegungsabläufe erlaubt.

Eine derartig ausgestaltete Kniegelenk-Endoprothese kann dann mit einer herkömmlichen Femurkomponente 2 kombiniert werden, welche auf der Gleitfläche 9 artikuliert. Die Kniegelenk-Endoprothese 1 weist dann in Extension eine hohe Stabilität auf und kann sich mit zunehmender Flexion medial um einen sich nur wenig anterio-posterior verschiebbaren Punkt 17 auf der medialen tibialen Gleitfläche 9b, welcher auf der Schnittebene 15 in Fig. 1B markiert ist, drehen, während die tibiale Innenrotation ungehindert einsetzt. Die Femurkomponente 2 kann sich mit zunehmender Flexion auf der lateralen tibialen Gleitfläche 9a entlang der in Fig. 1B mit 18 bezeichneten gekrümmten Linie nach posterior verschieben und gleichzeitig nach dorsal-distal absenken, was eine ungehinderte Innenrotation des Schienbeins, verbunden mit einer synchronen Außenrotation des Femurs, zuläßt und so der normalen Kinematik in tiefer Flexion entsprechend einzelne Weichteile weniger spannt und einen größeren Bewegungsumfang erlaubt.

Die laterale Gleitfläche 9a des Zwischenelements 3 ist derart ausgestaltet, dass das Drehzentrum der lateralen gekrümmten konkav-flach-konvexen Gleitfläche 9a in der medialen Gleitfläche 9b im Bereich 17 liegt. In der lateralen Gleitfläche 9a ändert sich der dorsale Krümmungsradius sowie die Höhe der Lauffläche in Funktion der Knieflexion und Innenrotation der Tibia.

Entsprechende Schnittprofile sind in den Fig. 2B bis 2D dargestellt. Dabei weisen die Ausführungsbeispiele gemäß Fig. 2B und 2D jeweils drei Zonen 19 mit unterschiedlichen Krümmungsradien auf, während das Ausführungsbeispiel gemäß Fig. 2C zwei Zonen 19 mit unterschiedlichen Krümmungsradien umfaßt. Die Krümmungsradien verlaufen dabei jeweils abnehmend von anterior nach posterior, wobei anterior und posterior in den Fig. 2 und 3 mit A und P bezeichnet sind.

Dabei bezeichnen die senkrechten Linien in den Fig. 2B bis 2D Wendepunkte 20, in welchen der Krümmungsradius einer Zone 19 in den Krümmungsradius einer angrenzenden Zone 19 wechselt. Die Krümmungsradien verhalten sich dabei stetig. Dementsprechend sind in Fig. 2B und 2D zwei Wendepunkte 20, in Fig. 2C ein Wendepunkt 20 des Krümmungsradius zu sehen.

In Extension weisen die mediale Gleitfläche 9b und die laterale Gleitfläche 9a eine hohe Kongruenz zur Femurkomponente 2 auf, lateral vor allem im anterioren Bereich. Die flache und/oder konvex abfallende laterale Gleitfläche 9a ist so ausgestaltet, dass die Innenrotation der Tibia um das mediale Zentrum 17 in der medialen Gleitfläche 9b erfolgt, während das sog. "Roll-back" des Femur nach dorsal/distal vorwiegend auf der lateralen Gleitfläche 9a erfolgt, wobei eine Drehung um das mediale Zentrum 17 erfolgt.

Somit wird eine Gleitfläche 9 angestrebt, welche eine konkave mediale Gleitfläche 9b und eine ventral konvex, dorsal konvex abfallende laterale Gleitfläche 9a aufweist.

Die konkave mediale Gleitfläche 9b weist in Extension eine hohe Kongruenz zur Femurkomponente 2 auf. Der translatorische Freiheitsgrad der Femurkomponente 2 nach anterior wird durch die anterior erhöhte Gleitfläche 9b sowie die Reaktionskraft der eingreifenden Weichteilstrukturen kontrolliert und begrenzt, wodurch ein Gleiten der Femurkomponente 2 nach anterior kontrolliert und eingeschränkt wird. Umgekehrt sind der translatorische Freiheitsgrad der Femurkomponente 2 nach posterior durch die posterior erhöhte Gleitfläche 9b sowie die Reaktionskraft der eingreifenden Weichteilstrukturen begrenzt und dadurch ein Gleiten der Femurkomponente 2 nach posterior ebenfalls eingeschränkt, wie aus Fig. 3 ersichtlich.

Die konkave mediale Gleitfläche 9b weist in Flexion ebenfalls eine hohe Kongruenz zur Femurkomponente 2 auf, die jedoch kleiner als in Extension ist, welche die Femuraußenrotation sowie die Bewegung der Femurkomponente 2 auf der lateralen Gleitfläche 9a nach dorsal-distal um den sich in der medialen Gleitfläche 9b befindlichen und sich anterio-posterior wenig, beispielsweise nur um 0-3mm verschiebenden Drehpunkt 17 zuläßt.

Die laterale Gleitfläche 9a ist demgegenüber konkav-flachkonvexer ausgebildet. In Extension ist der translatorische Freiheitsgrad der Femurkomponente 2 nach anterior durch die anterior erhöhte Gleitfläche 9a sowie die Reaktionskraft der eingreifenden Weichteilstrukturen begrenzt und dadurch ein Gleiten der Femurkomponente 2 nach anterior kontrolliert und einschränkt. Den translatorische Freiheitsgrad der Femurkomponente 2 nach posterior ist durch die konvexe Form der Gleitfläche 9a zwar zulässig, jedoch wird diese Bewegung durch die eintretende Drehbewegung des Femurs um den medialen Drehpunkt 17 und den dadurch gespannten femoralen Bandapparat, unterstützt durch die hohe Kongruenz in der medialen Gleitfläche 9b, kontrolliert und einschränkt. Die mediale Gleitfläche 9b ist somit für Stabilität hauptverantwortlich, während die laterale Gleitfläche 9a für die Beweglichkeit und Führung zuständig ist.

Die laterale Gleitfläche 9a ist in Flexion durch eine in den Ausführungsbeispielen nach dorsal und distal abfallende, konvexe, flache und/oder konkave Gleitfläche 9a gekennzeichnet, die die physiologische femorale Außenrotation und entsprechend die tibiale Innenrotation zuläßt und begünstigt, was einer physiologischeren Kinematik entspricht. Nach dorsal-distal nimmt durch die konvexe Gestalt die dorsale tibiale Neigung zu und somit die Höhe ab. Dadurch verschiebt sich mit zunehmendem femoralen "Roll-back" der Kontaktpunkt zwischen Femurkomponente 2 und Zwischenelement 3 nach distal.

Beide Gleitflächen 9a und 9b der modularen Gleitfläche 9 weisen in einem Frontalschnitt, in Fig. 1B durch eine punktgestrichelte Linie angedeutet, durch die Gleitfläche 9a, 9b eine konkave Form auf.

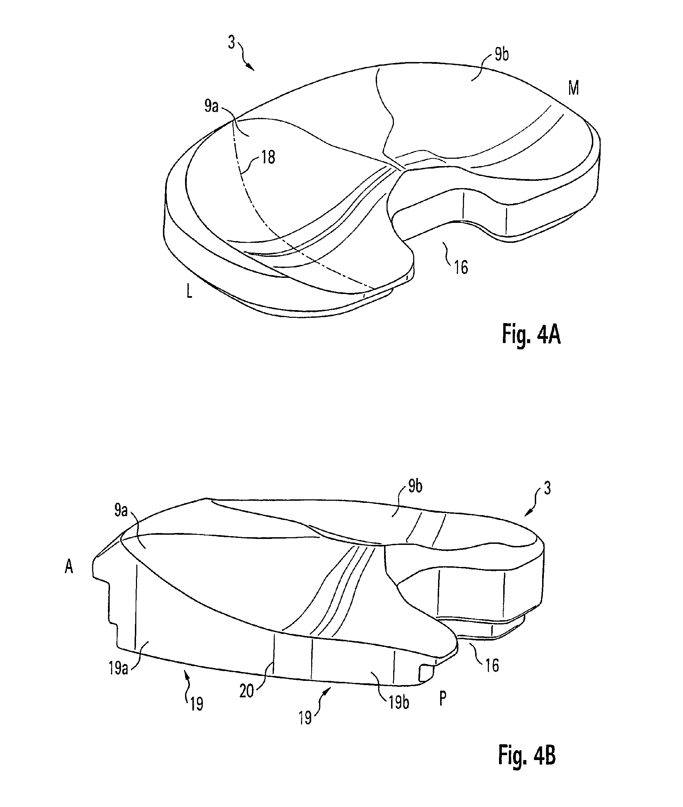

Die Fig. 4A und 4B zeigen zur Verdeutlichung des Sachverhalts eine schematische, perspektivische Darstellung bzw. eine perspektivische, teilgeschnittene Ansicht eines erfindungsgemäß ausgestalteten Zwischenelements gemäß Fig. 1B. Die gestrichelte Linie 18 in Fig. 4A korrespondiert dabei zu der gekrümmten Linie 18 in Fig. 1B. In der teilgeschnittenen Ansicht gemäß Fig. 4B ist eine Schnittfläche durch die laterale Gleitfläche 9a entlang der Linie 18 sichtbar, welche ein erfindungsgemäß ausgestaltetes gekrümmtes Profil gemäß Fig. 2C aufweist. Die Zonen 19, 19a, 19b und der im Ausführungsbeispiel eine Wendepunkt 20 sind deutlich erkennbar.

Das Zwischenelement 3 kann dabei aus Polymeren, keramischen und/oder metallischen Werkstoffen hergestellt sein. Besonders bevorzugt ist dabei das spezielle Polyethylen UHMWPE.

Vorteile der erfindungsgemäß ausgestalteten Kniegelenk-Endoprothese mit einem gemäß vorstehenden Ausführungen ausgebildeten Zwischenelement 3 sind insbesondere ein größerer Kniebewegungsumfang (range of motion), eine Verringerung des Impingment Risikos zwischen der Femurkomponente 2 und der Tibiakomponente 4, die physiologischere Kinematik durch weniger eingeschränkte Innenrotation der Tibia und Außenrotation des Femurs begünstigt durch Absinken der lateralen Gleitfläche 9a nach dorsal-distal in tiefer Flexion, dadurch bessere Langzeitresultate und durch physiologischer beanspruchte Weichteilstrukturen das Vermeiden derer Überlastung, was folglich die Reduktion chronischer Schmerzen bewirken kann.

Die Erfindung ist nicht auf die dargestellten Ausführungsbeispiele beschränkt, sondern für eine Vielzahl von weiteren Ausgestaltungsmöglichkeiten von Kniegelenk-Endoprothesen 1, insbesondere auf kreuzbanderhaltende und kreuzbandopfernde sowohl gekoppelte Total-Knieprothesen 1 mit Femurkomponente 2 und Tibiakomponente 4 anwendbar. Alle Merkmale der Erfindung sind dabei beliebig miteinander kombinierbar.

## Patentansprüche

1. Kniegelenk-Endoprothese (1), zum Ausbilden einer Gelenkverbindung zwischen einem Femurknochen und einem Tibiaknochen, mit einer Femurkomponente (2), die mit dem Femurknochen verbindbar ist, einer Tibiakomponente (4), die mit dem Tibiaknochen verbindbar ist, und mit einem zwischen der Tibiakomponente (4) und der Femurkomponente (2) gelagerten Zwischenelement (3), welches mit einer modularen Gleitfläche (9), umfassend eine laterale Gleitfläche (9a) und eine mediale Gleitfläche (9b), an der Femurkomponente (2) anliegt, und wobei mit der Tibiakomponente (4) lösbar verbindbar ist und die laterale Gleitfläche (9a) und die mediale Gleitfläche (9b) des Zwischenelements (3) unterschiedliche sagittale Querschnittsprofile aufweisen,
**dadurch gekennzeichnet,**
**daß** die laterale Gleitfläche (9a) zumindest zwei Zonen (19) aufweist und das sagittale Querschnittsprofil der lateralen Gleitfläche (9a) in zumindest einer der Zonen (19) flach oder konvex ausgebildet ist.

2. Kniegelenk-Endoprothese nach Anspruch 1,
**dadurch gekennzeichnet,**
**daß** das sagittale Querschnittsprofil der medialen Gleitfläche (9b) zumindest stückweise konkav geformtist.

3. Kniegelenk-Endoprothese nach Anspruch 1 oder 2,
**dadurch gekennzeichnet,**
**daß** in dem Krümmungsradius der lateralen Gleitfläche (9a) zumindest ein Wendepunkt (20) zwischen den zumindest zwei Zonen (19) vorgesehen ist.

4. Kniegelenk-Endoprothese nach einem der Ansprüche 1 bis 3,
**dadurch gekennzeichnet,**
**daß** die Anzahl der Zonen (19) zwei beträgt.

5. Kniegelenk-Endoprothese nach Anspruch 4,
**dadurch gekennzeichnet,**
**daß** eine anteriore Zone (19a) einen konkaven Krümmungsradius und eine posteriore Zone (19b) einen konvexen Krümmungsradius aufweist.

6. Kniegelenk-Endoprothese nach Anspruch 4 oder 5,
**dadurch gekennzeichnet,**
**daß** der konkave und der konvexe Krümmungsradius der Zonen (19a, 19b) an dem Wendepunkt (20) stetig ineinander übergehen.

7. Kniegelenk-Endoprothese nach einem der Ansprüche 1 bis 6,
**dadurch gekennzeichnet,**
**daß** die Anzahl der Zonen (19) drei beträgt.

8. Kniegelenk-Endoprothese nach Anspruch 7,
**dadurch gekennzeichnet,**
**daß** eine anteriore Zone (19a) einen konkaven Krümmungsradius und eine posteriore Zone (19b) einen konvexen Krümmungsradius aufweist.

9. Kniegelenk-Endoprothese nach Anspruch 7 oder 8,
**dadurch gekennzeichnet,**
**daß** eine zwischen der anterioren und der posterioren Zone (19a, 19b) ausgebildete mittlere Zone (19c) flach oder konkav ausgebildet ist.

10. Kniegelenk-Endoprothese nach einem der Ansprüche 7 bis 9,
**dadurch gekennzeichnet,**
**daß** die Krümmungsradien der drei Zonen (19a, 19c, 19b) an dazwischenliegenden Wendepunkten (20) stetig ineinander übergehen.

11. Kniegelenk-Endoprothese nach einem der Ansprüche 1 bis 10,
**dadurch gekennzeichnet,**
**daß** das Zwischenelement (3) aus einem Polymer und/oder einem keramischen und/oder metallischen Werkstoffen hergestellt ist.

12. Kniegelenk-Endoprothese nach Anspruch 11,
**dadurch gekennzeichnet,**
**daß** das Polymer Polyethylen, insbesondere UHMWPE, ist.

## Claims

1. Knee joint endoprosthesis (1) for forming an articulation between a femur and a tibia, with a femur component (2) which can be connected to the femur, a tibia component (4) which can be connected to the tibia, and with an intermediate element (3) which is mounted between the tibia component (4) and the femur component (2), lies against the femur component (2) with a modular sliding surface (9) comprising a lateral sliding surface (9a) and a medial sliding surface (9b), and can be connected in a releasable manner to the tibia component (4), and the lateral sliding surface (9a) and the medial sliding surface (9b) of the intermediate element (3) have different sagittal cross-sectional profiles
**characterised in**
**that that** the lateral sliding surface (9a) has at least two zones (19) and the sagittal cross-sectional profile of the lateral sliding surface (9a) is of flat or convex formation in at least one of the zones (19).

2. Knee joint endoprosthesis according to Claim 1,
**characterised in**
**that** the sagittal cross-sectional profile of the medial sliding surface (9a) is shaped so as to be concave at least in parts.

3. Knee joint endoprosthesis according to Claim 1 or 2,
**characterised in**
**that** at least one turning point (20) between the at least two zones (19) is provided in the radius of curvature of the lateral sliding surface (9a).

4. Knee joint endoprosthesis according to one of Claims 1 to 3,
**characterised in**
**that** there are two zones (19).

5. Knee joint endoprosthesis according to Claim 4,
**characterised in**
**that** an anterior zone (19a) has a concave radius of curvature and a posterior zone (19b) has a convex radius of curvature.

6. Knee joint endoprosthesis according to Claim 4 or 5,
**characterised in**
**that** the concave and the convex radii of curvature of the zones (19a, 19b) pass continuously into one another at the turning point (20).

7. Knee joint endoprosthesis according to one of Claims 1 to 6,
**characterised in**
**that** there are three zones (19).

8. Knee joint endoprosthesis according to Claim 7,
**characterised in**
**that** an anterior zone (19a) has a concave radius of curvature and a posterior zone (19b) has a convex radius of curvature.

9. Knee joint endoprosthesis according to Claim 7 or 8,
**characterised in**
**that** a central zone (19c) formed between the anterior and the posterior zones (19a, 19b) is of flat or concave formation.

10. Knee joint endoprosthesis according to one of Claims 7 to 9,
**characterised in**
**that** the radii of curvature of the three zones (19a, 19c, 19b) pass continuously into one another at turning points (20) lying between them.

11. Knee joint endoprosthesis according to one of Claims 1 to 10,
**characterised in**
**that** the intermediate element (3) is manufactured from a polymer and/or a ceramic and/or metallic material(s).

12. Knee joint endoprosthesis according to Claim 11,
**characterised in**
**that** the polymer is polyethylene, in particular UHMWPE.

## Revendications

1. Endoprothèse de l'articulation du genou (1), destinée à réaliser une liaison articulée entre un os de fémur et un os de tibia, comprenant un composant de fémur (2), qui peut être relié à l'os de fémur, un composant de tibia (4), qui peut être relié à l'os de tibia, et comprenant un élément intermédiaire (3) qui est monté entre le composant de tibia (4) et le composant de fémur (2), et s'appuie sur le composant de fémur (2) avec une surface de glissement modulaire (9) englobant une surface de glissement latérale (9a) et une surface de glissement médiale (9b), l'élément intermédiaire pouvant être relié de manière amovible avec le composant de tibia (4), et la surface de glissement latérale (9a) et la surface de glissement médiale (9b) de l'élément intermédiaire (3) présentant des profils de section sagittale différents,
**caractérisée**
**en ce que** la surface de glissement latérale (9a) présente au moins deux zones (19), et le profil de section sagittale de la surface de glissement latérale (9a) est, dans l'une au moins des zones (19), de configuration plane ou convexe.

2. Endoprothèse de l'articulation du genou selon la revendication 1,
**caractérisée**
**en ce que** le profil de section sagittale de la surface de glissement médiale (9b) est, au moins sur certaines parties, de forme concave.

3. Endoprothèse de l'articulation du genou selon la revendication 1 ou la revendication 2,
**caractérisée**
**en ce que** dans le rayon de courbure de la surface de glissement latérale (9a) est prévu au moins un point d'inflexion (20) entre lesdites au moins deux zones (19).

4. Endoprothèse de l'articulation du genou selon l'une des revendications 1 à 3,
**caractérisée**
**en ce que** le nombre des zones (19) est de deux.

5. Endoprothèse de l'articulation du genou selon la revendication 4,
**caractérisée**
**en ce qu'**une zone antérieure (19a) présente un rayon de courbure concave, et une zone postérieure (19b) présente un rayon de courbure convexe.

6. Endoprothèse de l'articulation du genou selon la revendication 4 ou la revendication 5,
**caractérisée**
**en ce que** les rayons de courbure concave et convexe des zones (19a, 19b) se raccordent de manière continue au niveau du point d'inflexion (20).

7. Endoprothèse de l'articulation du genou selon l'une des revendications 1 à 6,
**caractérisée**
**en ce que** le nombre des zones (19) est de trois.

8. Endoprothèse de l'articulation du genou selon la revendication 7,
**caractérisée**
**en ce qu'**une zone antérieure (19a) présente un rayon de courbure concave, et une zone postérieure (19b) présente un rayon de courbure convexe.

9. Endoprothèse de l'articulation du genou selon la revendication 7 ou la revendication 8,
**caractérisée**
**en ce qu'**une zone centrale (19c) formée entre la zone antérieure et la zone postérieure (19a, 19b), est de configuration plane ou concave.

10. Endoprothèse de l'articulation du genou selon l'une des revendications 7 à 9,
**caractérisée**
**en ce que** les rayons de courbure des trois zones (19a, 19c, 19b) se raccordent de manière continue au niveau de points d'inflexion (20) situés entre elles.

11. Endoprothèse de l'articulation du genou selon l'une des revendications 1 à 10,
**caractérisée**
**en ce que** l'élément intermédiaire (3) est fabriqué en un polymère et/ou des matériaux céramiques et/ou métalliques.

12. Endoprothèse de l'articulation du genou selon la revendication 11,
**caractérisée**
**en ce que** le polymère est du polyéthylène, notamment du UHMWPE (polyéthylène à poids moléculaire «ultra-haut»).
